# EUROPEAN PATENT SPECIFICATION

(11) **EP 0 331 085 B1**
(45) Date of publication and mention of the grant of the patent: **07.09.1994**
(21) Application number: 89103425.8
(22) Date of filing: 27.02.1989
(51) Int. Cl.: C07C 65/21, C07C 65/24, C07C 59/68, C07C 59/70, C07C 59/90

(54) **Polyoxyalkylene ether derivatives of carboxylic acid salts and esters**
Polyoxyalkylenätherderivate von Carbonsäuresalzen und -estern
Dérivés polyoxyalkylène étheriques de sels et d'esters d'acides carboxyliques

(30) Priority: 29.02.1988 IT 1957688
(43) Date of publication of application: 06.09.1989
(73) Proprietor: Montefibre S.p.A., Milan (IT)
(72) Inventor: Bastioli, Catia, Dr., I-28100 Novara (IT); Garlisi, Salvatore, Dr., I-13100 Vercelli (IT); Fornara, Dario, Dr., I-28100 Novara (IT); Bellotti, Vittorio, I-28010 Fontaneto d'Agogna Novara (IT)
(74) Representative: Weinhold, Peter, Dr.

(56) References cited:
- DE-A- 3 637 084
- PATENT ABSTRACTS OF JAPAN, vol. 12, no. 333 (C-526)[3180], 8th September 1988; & JP-A-63 96 152

## Description

The present invention relates to polyoxyalkylene ether derivatives having organic end groups of general formula:

-O-Ar-(X)ₚCOOM (II)

and more particularly to compounds having general formula (I) 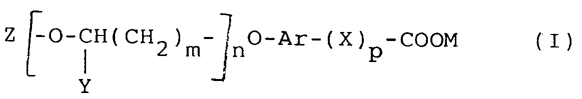
wherein:
- M: is an alkali or alkaline earth metal or a C₁-C₁₈ alkyl group (in the case of alkaline earth metals M represents half an equivalent of the bivalent ion);
- X: is a group selected from -CH₂-, 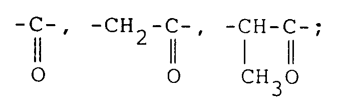
- p: is 0 or 1;
- Ar: is a single or condensed aromatic ring of from 6 to 14 carbon atoms, optionally substituted with one or more groups selected from halogen atoms, C₁-C₈ alkyl, C₁-C₈ alkoxy, or nitro groups; or Ar represents two or more aromatic nuclei, linked either by a simple covalent bond or by an -O-, -S-, SO₂-, -SO-, -CO-, -CS-, -NH- group;
- Y: is H or CH₃;
- m: is 1 when Y is CH₃, or is an integer of from 1 to 5, preferably 1 to 3 (including both extremes) when Y is H;
- n: is a number ranging from 1 to 500 (including both extremes);
- Z: is selected from H; C₁-C₁₈ alkyl; benzyl; allyl; 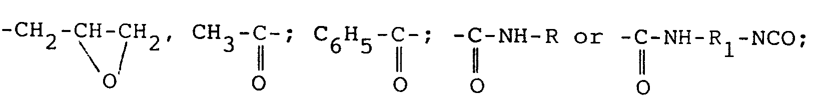 wherein R is an unsubstituted phenyl group or a phenyl group substituted with one or more halogen atoms (e.g. F and Cl) or a C₁₀-C₄₀ alkyl group; and R₁ represents an unsubstituted phenylene group or a phenylene group substituted with one or more halogen atoms (e.g. F and Cl) or a C₁₀-C₄₀ alkylene group.

Preferred meanings for the various groups and indices mentioned above are:
- M:: Na, K, Li, Ca and Mg, particularly Na and K; C₁₋₁₂-(particularly C₁₋₆-)alkyl groups, such as methyl, ethyl, propyl and butyl;
- Ar:: phenylene, naphthylene, biphenylene, optionally substituted with one or more, particularly 1 or 2 groups selected from F, Cl, Br, I (particularly F and Cl), C₁₋₈-(preferably C₁₋₄)alkyl groups, (such as methyl, ethyl, n- and i-propyl, n-, sek.-, i- and tert.-butyl), C₁₋₈-(preferably C₁₋₄-)alkoxy groups (such as methoxy, ethoxy, propoxy and butoxy) and NO₂; also preferred are phenylene groups linked by -O-, -S-, -SO₂-, -SO-, -CO-, -CS- and -NH- groups;
- n:: an integer ranging from about 10 to about 250, particularly from about 20 to about 150;
- Z:: H, C₁₋₁₀-(particularly C₁₋₆-)alkyl groups (such as methyl, ethyl, propyl, butyl, pentyl and hexyl), acetyl, glycidyl, benzyl, alkyl, CONHR₃ and CONHR₄NCO;
- R:: unsubstituted phenyl, C₁₀₋₃₀-(particularly C₁₀₋₂₀-)alkyl groups;
- R₁:: unsubstituted phenylene, C₁₀₋₃₀-(particularly C₁₀₋₂₀)alkylene groups.

Such compounds, due to their particular structure, can be used as plasticizers and as nucleating agents for polyester resins, because the structural units which confer such properties are combined in the same molecule.

Alkali metal salts of carboxylic acids and in particular of benzoic acid as nucleating agents for PET are known from the prior art (see for instance US-A-4 486 561 and JP-A-59/230048).

However, such formulations generally require the additional use of other additives, and in particular of plasticizers, in order to affect the crystallization temperature in the molten state, the cold crystallization temperature and the glass transition temperature of the amorphous phase.

The compounds of general formula (I) can be looked upon as saline nucleating agents, which are chemically bound to a polymeric oxyalkylene chain.

By using these compounds the final properties for a good molding of the polyester resin can be obtained because, besides affording the required degree of plasticization, due to the presence of the polyoxyalkylene chain, this chain, soluble in the resin, promotes the dispersion of the nucleating agent, with formation of a homogeneous microspherulitical crystalline structure.

Moreover the OH terminated end of the polyoxyalkylene chain may be functionalized by ether, ester, epoxide or urethane groups and, therefore, allows adaptability to a wide variety of applications, according to the required final properties.

Examples of products according to general formula (I), are the ones in which Z, Y, Ar, X, M, m and n have the meanings outlined in the following Table.

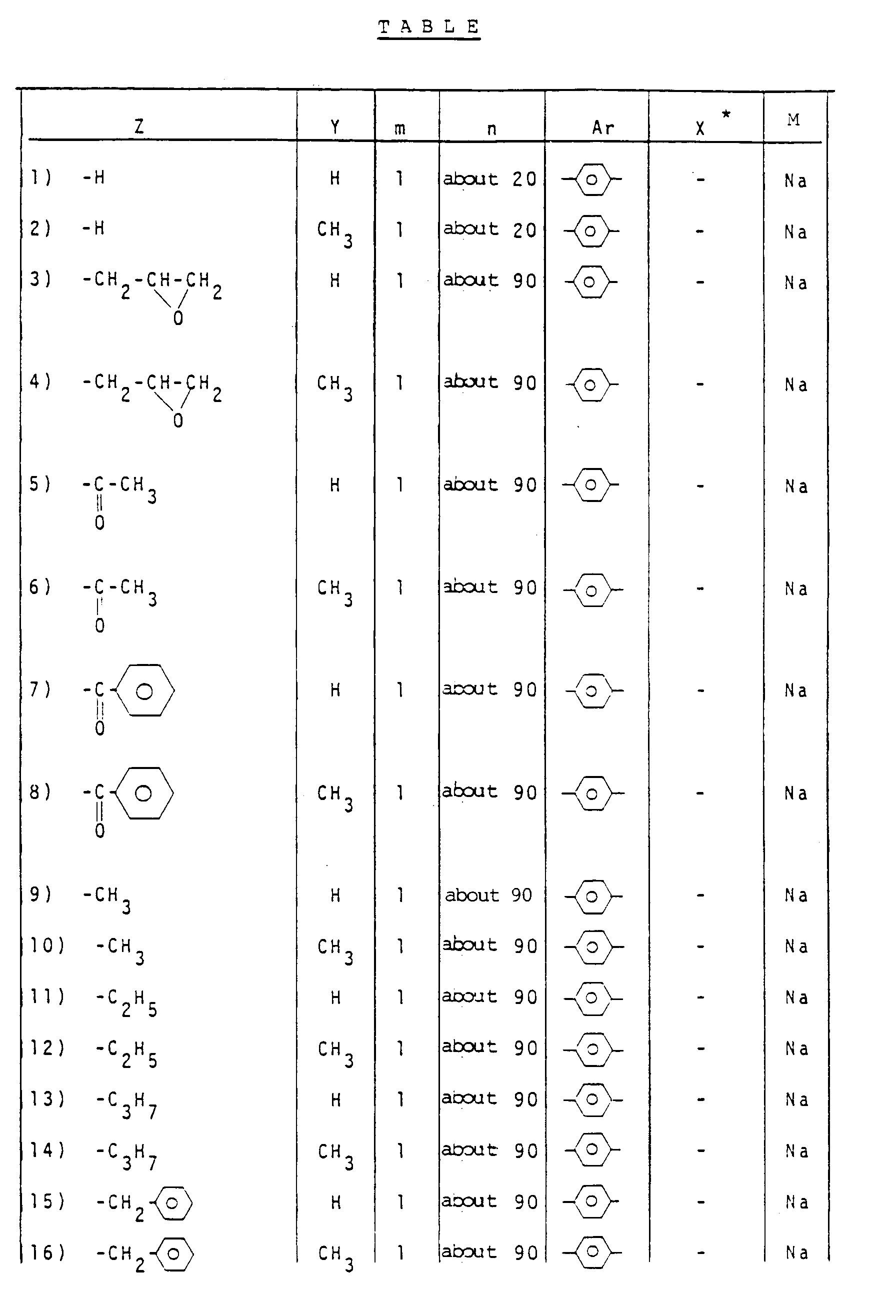

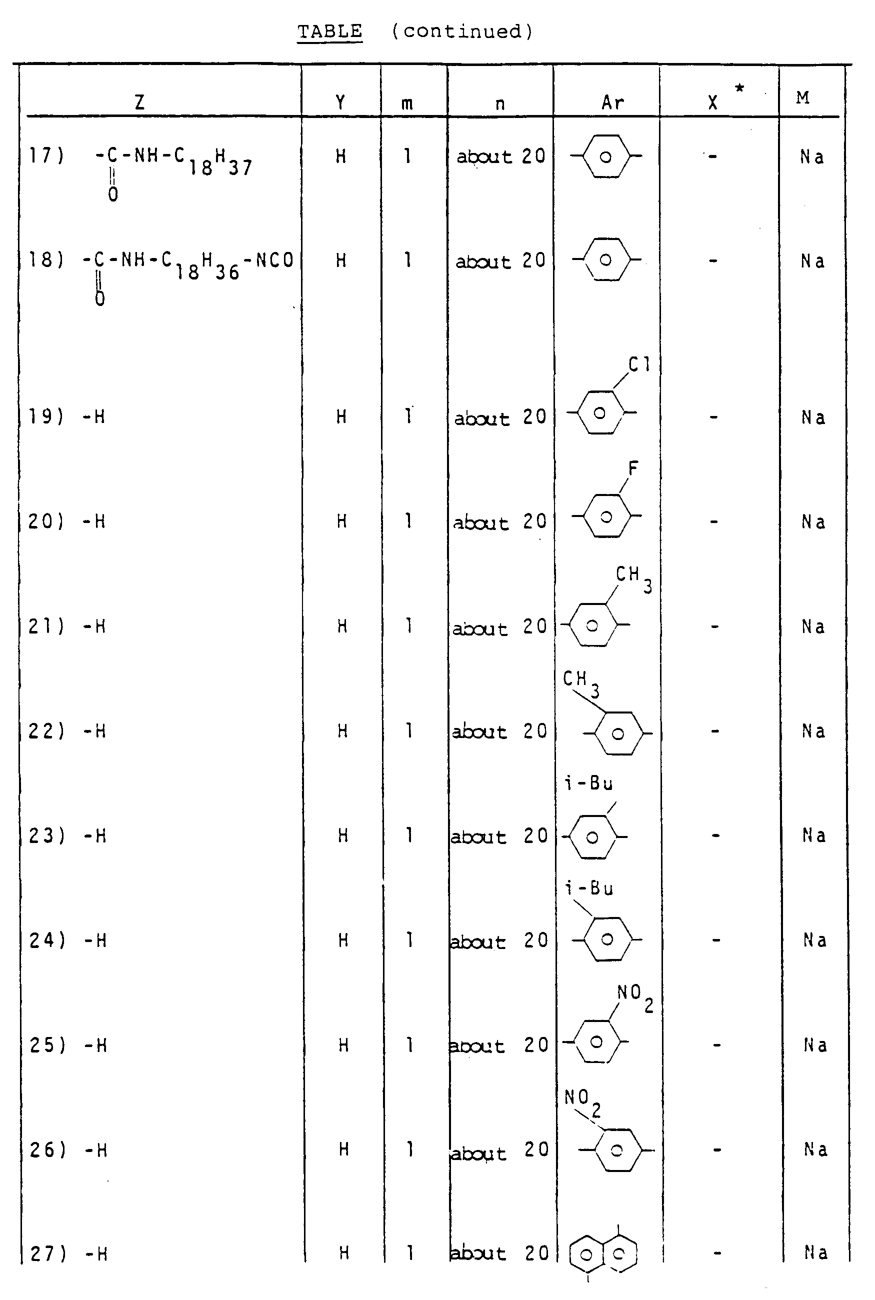

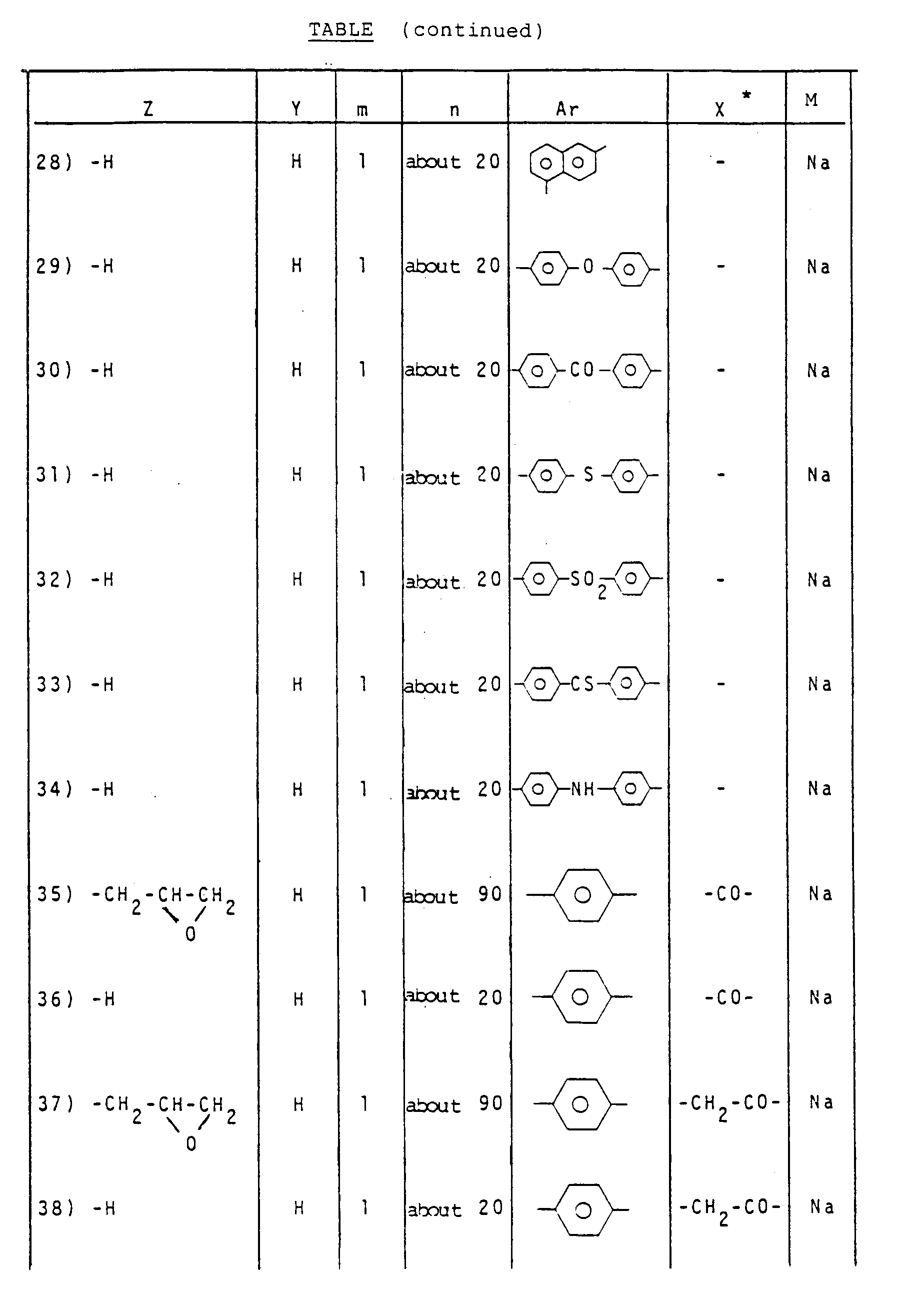

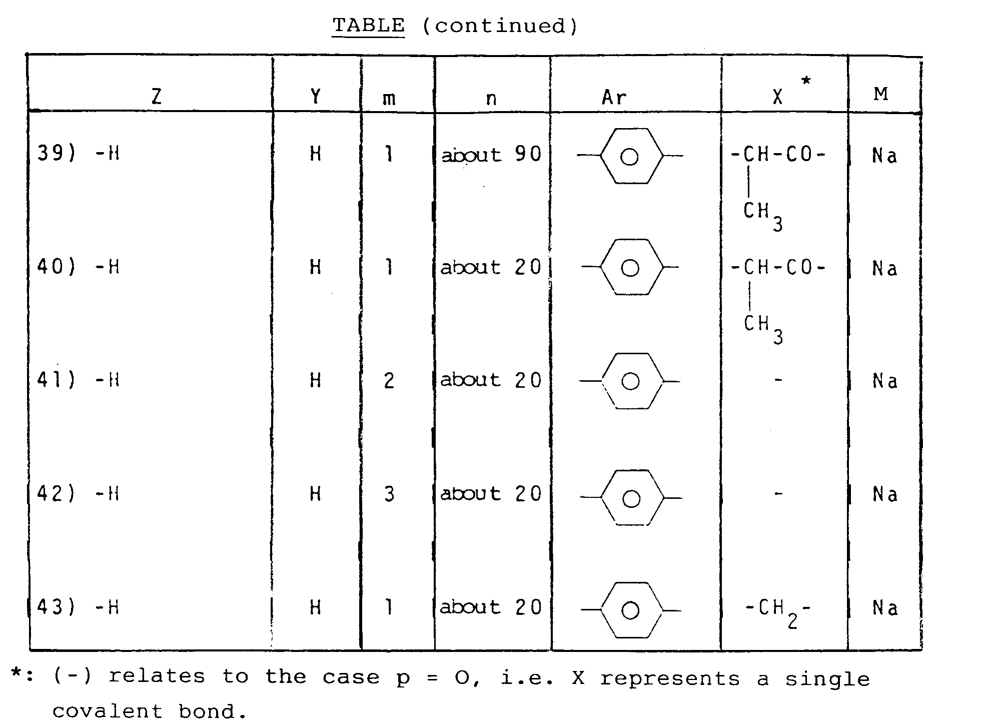

Although all these products can be used as nucleating agents and as plasticizers for polyester resins, in particular the ones based on PET, particularly preferred for this purpose are products of general formula (I) wherein Ar is 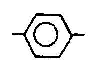
and X represents a single covalent bond, i.e. the polyoxyalkylene ether derivatives having as their end-groups alkali and alkaline earth metal salts and esters of p-hydroxybenzoic acid.

The compounds having general formula (I) are generally prepared by conventional methods, starting from an alkyl ester of an acid having formula (III):

HO-Ar-(X)-ₚCOOH (III)

wherein Ar, X and p have the meanings specified above.

In a first stage of the preparation, said esters are reacted in an autoclave with an alkylene oxide in the presence of alkali at a temperature of 130°C and at a pressure of up to about 3.04 bar (3 atmospheres).

Afterwards the thus obtained product is saponified with an alkali metal hydroxide in order to obtain the salt of the acid, if the product is desired in this form.

The third stage of the preparation comprises the functionalization of the OH end-group of the polyoxyalkylene chains according to known techniques.

A few examples concerning the preparation of products of general formula (I) are given hereinafter, merely by way of illustration, but not of limitation.

The numbers of ethylene oxide units incorporated into the compounds, as given hereinafter, are naturally average values. By changing the ratios of ethylene oxide to the remaining reagents, according to known methods, it is possible to insert an oxyethylene chain of any desired length.

### Example 1

### Preparation of ethyl para-(hydroxy-polyethyleneoxy)-benzoate (about 90 units of EO)

A mixture of 185.5 g of ethyl para-hydroxybenzoate, 6.2 g of potassium hydroxide flakes and 425.0 g of xylene was heated under stirring at 100°C until a slightly cloudy solution was obtained.

Then the solution was sucked into an autoclave and the system was freed of oxygen by repeatedly applying vacuum and purging with nitrogen gas.

After having brought the temperature to 130°C under stirring, the addition of ethylene oxide (total amount of 4392.5 g) was commenced, keeping the temperature at 130°C and the pressure at about 3.04 bar (3 atmospheres). The reaction time was about 6 hours

When the addition of ethylene oxide was complete, after exhaustion of said ethylene oxide, the system was kept under vacuum for about 15 minutes; then the mixture was cooled to 90°C, the autoclave was discharged and the reaction system was neutralized with glacial acetic acid (6.6 g).

The thus obtained reaction mixture was conveyed into a 6 l reactor, equipped with anchor stirrer, thermometer and Liebig condenser. After heating to about 100°C, vacuum was applied (6666 Pa) (50 mm Hg).

About 350 g of xylene were distilled off (the remainder of the amount that had been introduced had already been removed in the vacuum stage following the ethoxylation).

Upon completion of the distillation, the pressure was increased to normal and the mixture was cooled to about 80°C and discharged.
Obtained were about 4700 g of ethyl para-(hydroxy-polyethyleneoxy)-benzoate, containing about 90 units of EO, having the following characteristics:

| | |
|---|---|
| - acid number: | 1.1 |
| - hydroxyl number: | 24.1 |
| - saponification number: | 13.8 |

### Example 2

### Preparation of sodium para-(hydroxy-polyethyleneoxy)-benzoate (about 90 units of EO)

500 g of ethyl para-(hydroxy-polyethylene)-benzoate (about 90 units of EO), obtained according to example 1, and 16.5 g of 30% sodium hydroxide were fed into a 1 l reactor equipped with anchor stirrer, thermometer and bubble condenser. After having increased the internal temperature to 80°C under stirring, the system was kept at this temperature for 1 hour.

After having replaced the bubble condenser by a Liebig condenser, vacuum was applied (6666 Pa) (50 mm/Hg). The reaction mixture was kept under these conditions for half an hour, during which time about 12 g of water and ethanol were distilled off. Thereafter atmospheric pressure was applied and the mixture was cooled to 50°C, and subsequently discharged. Obtained were about 500 g of a product having the following characteristics:

| | |
|---|---|
| - acid number: | 0.2 |
| - hydroxyl number: | 36.3. |

### Example 3

### Preparation of sodium para-(glycidoxy-polyethyleneoxy)benzoate (about 90 units of EO)

300 g of sodium para-(hydroxy-polyethyleneoxy)-benzoate (about 90 units of EO) obtained according to the procedure described in example 2 and 3.3 g of ROLCRIL ® S (lauryldimethylbenzyl ammonium chloride, 50% in water) were fed into a 0.5 l reactor equipped with anchor stirrer, dropping funnel, thermometer and bubble condenser.

After having brought the internal temperature to 60°C under stirring, 7.9 g of sodium hydroxide drops were added within 5 minutes: thereafter the system was kept under the same conditions for 1 hour.

Then 18 g of epichlorohydrin were added at such a rate that the whole amount was introduced within 1 hour, whereafter the reaction mixture was kept under the same conditions for another 6 hours.

Then the internal temperature was increased to 80°C, the bubble condenser was replaced by a Liebig condenser and vacuum was applied (6666 Pa) (50 mm Hg). The system was kept under these conditions for 1 hour, during which time a small amount of water distilled. Thereafter the reactor was unloaded. Obtained were about 320 g of a product having the following characteristics:

| | |
|---|---|
| appearance at 20°C: | yellow flakes |
| melting point: | + 55°C |
| acid number: | 0.4 |
| saponified acid number: | 14.4 |
| epoxide equivalent: | 1902.7 |
| NaCl (%): | 3.5 |
| H₂O (%): | 0.3 |

### Example 4

### Preparation of sodium para-(acetyloxy-polyethyleneoxy)benzoate (about 90 units of EO)

200 g of sodium para-(hydroxy-polyethyleneoxy)-benzoate (about 90 units of EO), obtained according to example 2, were fed into a 0.5 l reactor equipped with anchor stirrer, dropping funnel, thermometer and bubble condenser. After having brought the internal temperature to 95°C under stirring 15.3 g of acetic anhydride were added within 45 minutes. When the addition was complete, the reaction mixture was kept at 95°C and under stirring for 2 hours.

Then the bubble condenser was replaced by a Liebig condenser and vacuum was applied (6666 Pa) (50 mm Hg). The internal temperature was raised to 140°C. The acetic acid began to distill off at 130°C.

The system was kept at 140°C until the acid number of the mixture was ≦3 (the required time was about 2 hours). Then normal pressure was applied, the mixture was cooled to 60°C and discharged.
Obtained were about 205 g of a product, having the following characteristics:

| | |
|---|---|
| appearance at 20°C: | beige flakes |
| melting point: | 50°C |
| acid number: | 2.2 |
| hydroxyl number: | 0.5 |
| saponified acid number: | 14.0 |

### Example 5

### Preparation of sodium para-(benzyloxy-polyethyleneoxy)-benzoate (about 90 units of EO)

200 g of sodium para-(hydroxy-polyethyleneoxy)-benzoate (about 90 units of EO), obtained according to example 2 and 2.5 g of ROLCRIL ® S were fed into a 0.5 l reactor, equipped with anchor stirrer, dropping funnel, thermometer and bubble condenser. The internal temperature was brought to 60°C under stirring; then 5.6 g of sodium hydroxide drops were added within 5 minutes and the reaction mixture was kept at 60°C and under stirring for 1 h. Thereafter 19.2 g of benzyl chloride were added within 1 hour. The system was kept under the same conditions for further 6 hours and thereafter the internal temperature was brought to 80°C. The bubble condenser was replaced by a Liebig condenser and vacuum was applied (6666 Pa) (50 mm Hg). The system was kept under these conditions for 1 hour, during which time a small amount of water distilled. Thereafter the mixture was discharged.
Obtained were about 220 g of a product having the following characteristics:

| | |
|---|---|
| appearance at 20°C: | yellow flakes |
| melting point: | 51°C |
| acid number: | 0.1 |
| saponified acid number: | 13.9 |
| hydroxyl number: | 17.4 |
| NaCl (%) | 4.1 |

### Example 6

### Preparation of ethyl para-(hydroxy-polyethyleneoxy)-benzoate (about 20 units of EO)

A mixture of 368.1 g of ethyl para-hydroxy-benzoate, 9.0 g of potassium hydroxide flakes and 858.6 g of xylene was heated under stirring at about 100°C, in order to obtain a slightly cloudy solution. Then the solution was sucked into an autoclave and the system was freed of oxygen by repeatedly applying vacuum and purging with nitrogen gas.

After having brought the temperature to 130°C under stirring, the addition of ethylene oxide (total amount of 1773.3 g) was commenced, keeping the temperature at 130°C and the pressure at about 3.04 bar (3 atmospheres). The reaction time was about 3 hours. After the addition of ethylene oxide was complete, after exhaustion of said ethylene oxide, the system was kept under vacuum for 15 minutes. The resulting mixture was cooled to 90°C, the autoclave was unloaded and the reaction system was neutralized with glacial acetic acid (9.6 g).

The thus obtained reaction mixture was conveyed into a 4 l reactor, equipped with anchor stirrer, thermometer and Liebig condenser. After heating to about 100°C, vacuum was applied (6666 Pa) (50 mm Hg).

About 590 g of xylene were distilled off (the remainder of the amount that had been loaded had already been removed in the vacuum stage following the ethoxylation). Upon completion of the distillation normal pressure was applied and the resulting mixture was cooled to about 80°C and discharged. Obtained were about 2100 g of a product having the following characteristics:

| | |
|---|---|
| acid number: | 2.6 |
| saponification number: | 59.5 |
| hydroxyl number: | 61.1 |

### Example 7

### Preparation of sodium para-(hydroxy-polyethyleneoxy)-benzoate (about 20 units of EO)

220 g of ethyl para-(hydroxy-polyethyleneoxy)-benzoate (about 20 units of EO) obtained according to example 6 and 31.1 of 30% sodium hydroxide were fed into a 0.5 l reactor, equipped with anchor stirrer, thermometer and bubble condenser. After having brought the internal temperature to 80°C under stirring, this temperature was kept for 1 hour. The product became increasingly pasty, making stirring difficult. After 1 hour, the bubble condenser was replaced by a Liebig condenser and vacuum was applied (6666 Pa) (50 mm Hg). The reaction mixture was kept under this condition for 1 hour, during which time about 20 g of water and ethanol distilled. Thereafter normal pressure was applied, the mixture was cooled and the reactor was unloaded. Obtained were about 220 g of a product having the following characteristics:

| | |
|---|---|
| appearance at 20°C: | pasty mass |
| alkali number: | 0.7 |
| hydroxyl number: | 90.7 |

## Claims (Claims for the following Contracting State(s): AT, BE, DE, FR, GB, NL, SE)

1. Compounds of general formula (I): 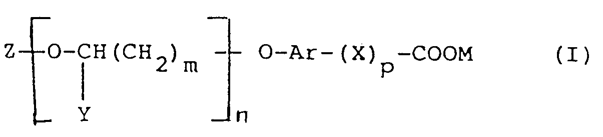 wherein
M is an alkali or half an equivalent of an alkaline earth metal or a C₁-C₁₈ alkyl group;
X is a group selected from 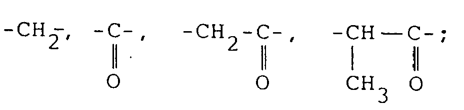 p is 0 or 1;
Ar is a single or condensed aromatic ring, having from 6 to 14 carbon atoms, optionally substituted with one or more groups selected from halogen atoms, C₁-C₈ alkyl, C₁-C₈ alkoxy, or nitro groups; or Ar represents 2 or more aromatic nuclei linked either by a simple covalent bond or by an -O-, -S-, -SO₂- -SO-, -CO-, -CS-, -NH- group;
Y is H or CH₃;
m is 1 when Y is CH₃;or is an integer of from 1 to 5 inclusive when Y is H;
n is a number ranging from 1 to 500 inclusive,
Z is selected from H; C₁-C₁₈ alkyl, benzyl, allyl, 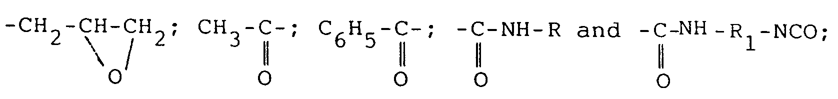 wherein R is an unsubstituted phenyl group or a phenyl group substituted with one or more halogen atoms or is a C₁₀-C₄₀ alkyl group; and R₁ represents unsubstituted phenylene or phenylene substituted with one or more halogen atoms or is a C₁₀-C₄₀ alkylene group.

2. Compounds according to claim 1 wherein p is O and Ar is a phenylene group.

3. Compounds according to any one of claims 1 and 2, wherein Z is H, 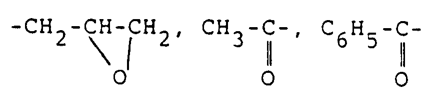 or an allyl group.

4. Compounds according to anyone of claims 1 to 3, wherein n is about 20.

5. Compounds according to anyone of claims 1 to 3, wherein n is about 90.

6. Sodium para-(hydroxy-polyethyleneoxy)-benzoate of formula 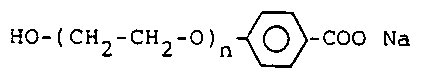 wherein n is about 90.

7. Sodium para-(glycidoxy-polyethyleneoxy)benzoate of formula 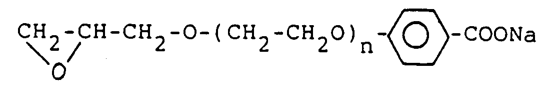 wherein n is about 90.

8. Sodium para-(acetyloxy-polyethyleneoxy)benzoate of formula 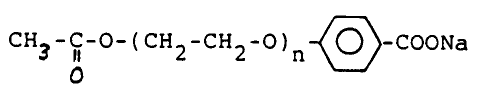 wherein n is about 90.

9. Sodium para-(benzyloxy-polyethyleneoxy)benzoate of formula 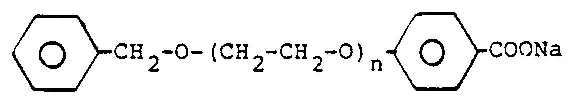 wherein n is about 90.

10. Sodium para-(hydroxy-polyethyleneoxy)benzoate of formula 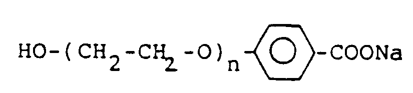 wherein n is about 20.

## Claims (Claims for the following Contracting State(s): ES)

1. Process for the preparation of compounds of general formula (I): 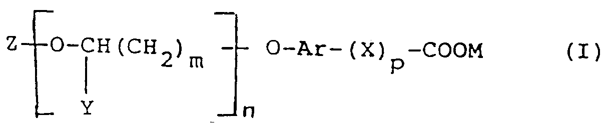 wherein
M is an alkali or half an equivalent of an alkaline earth metal or a C₁-C₁₈ alkyl group;
X is a group selected from -CH₂-, 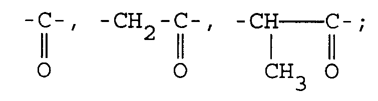
p is 0 or 1;
Ar is a single or condensed aromatic ring, having from 6 to 14 carbon atoms, optionally substituted with one or more groups selected from halogen atoms, C₁-C₈ alkyl, C₁-C₈ alkoxy, or nitro groups; or Ar represents 2 or more aromatic nuclei linked either by a simple covalent bond or by an -O-, -S-, -SO₂-, -SO-, -CO-, -CS-, -NH- group;
Y is H or CH₃;
m is 1 when Y is CH₃; or is an integer of from 1 to 5 inclusive when Y is H;
n is a number ranging from 1 to 500 inclusive,
Z is selected from H; C₁-C₁₈ alkyl, benzyl, allyl, 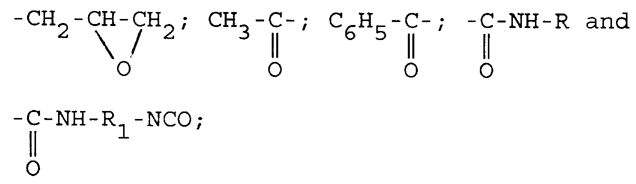 wherein R is an unsubstituted phenyl group or a phenyl group substituted with one or more halogen atoms or is a C₁₀-C₄₀ alkyl group; and R₁ represents unsubstituted phenylene or phenylene substituted with one or more halogen atoms or is a C₁₀-C₄₀ alkylene group, characterized in that an alkyl ester of an acid having formula (III):
HO-Ar-(X)-ₚCOOH (III)
wherein Ar, X and p have the meanings specified above, is reacted with an alkylene oxide in the presence of alkali at a temperature of 130°C and at a pressure of up to 3.04 bar (3 atmospheres), the thus obtained product is then saponified, if desired, with a metal hydroxide in order to obtain the salt of the acid, and the OH end-group of the polyoxyalkylene chain is subsequently functionalized, if desired.

2. Process according to claim 1 wherein p is O and Ar is a phenylene group.

3. Process according to any one of claims 1 and 2, wherein Z is H, 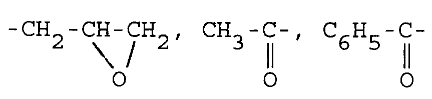 or an allyl group.

4. Process according to anyone of claims 1 to 3, wherein n is about 20.

5. Process according to anyone of claims 1 to 3, wherein n is about 90.

6. Process according to anyone of claims 1 to 5 for the preparation of sodium para-(hydroxy-polyethyleneoxy)-benzoate of formula 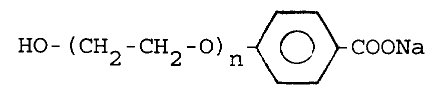 wherein n is about 90.

7. Process according to anyone of claims 1 to 5 for the preparation of sodium para-(glycidoxy-polyethyleneoxy)benzoate of formula 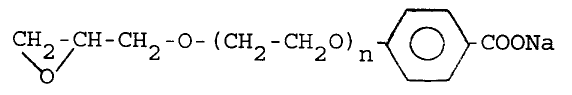 wherein n is about 90.

8. Process according to anyone of claims 1 to 5 for the preparation of sodium para-(acetyloxy-polyethyleneoxy)benzoate of formula 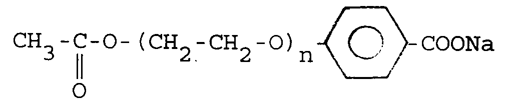 wherein n is about 90.

9. Process according to anyone of claims 1 to 5 for the preparation of sodium para-(benzyloxy-polyethyleneoxy)benzoate of formula 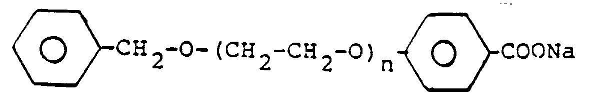 wherein n is about 90.

10. Process according to anyone of claims 1 to 5 for the preparation of sodium para-(hydroxy-polyethyleneoxy)benzoate of formula 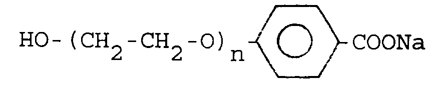 wherein n is about 20.

## Patentansprüche (Patentansprüche für folgende(n) Vertragsstaat(en): AT, BE, DE, FR, GB, NL, SE)

1. Verbindungen der allgemeinen Formel (I): 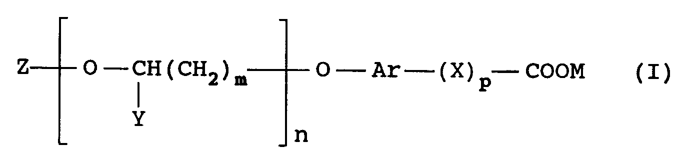 worin
M ein Alkalimetall oder ein halbes Äquivalent eines Erdalkalimetalls oder eine C₁-C₁₈-Alkyl-Gruppe ist;
X eine Gruppe ist, die ausgewählt ist aus -CH₂-, 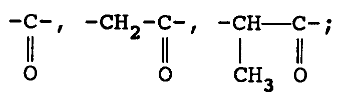
p = 0 oder 1 ist;
Ar ein einzelner oder ein kondensierter aromatischer Ring mit 6 bis 14 Kohlenstoffatomen ist, der wahlweise mit einer oder mehreren Gruppen substituiert ist, die ausgewählt sind aus Halogen-Atomen, C₁-C₈-Alkyl-, C₁-C₈-Alkoxy- oder Nitro-Gruppen; oder Ar für 2 oder mehrere aromatische Kerne steht, die entweder durch eine einfache kovalente Bindung oder durch eine -O-, -S-, -SO₂-, -SO-, -CO-, -CS- oder -NH- Gruppe miteinander verbunden sind;
Y H oder CH₃ ist;
m = 1 ist, wenn Y = CH₃ ist; oder eine ganze Zahl von 1 bis einschließlich 5 ist, wenn Y = H ist;
n eine Zahl im Bereich von 1 bis einschließlich 500 ist;
Z ausgewählt ist aus H; C₁-C₁₈-Alkyl, Benzyl, Allyl, 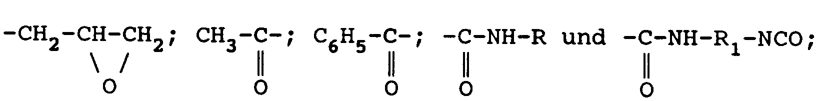 worin R eine unsubstituierte Phenyl-Gruppe ist oder eine Phenyl-Gruppe, die mit einem oder mehreren Halogen-Atomen substituiert ist, oder eine C₁₀-C₄₀-Alkyl-Gruppe ist; und worin R₁ unsubstituiertes Phenylen oder Phenylen bedeutet, welches mit einem oder mehreren Halogen-Atomen substituiert ist, oder eine C₁₀-C₄₀-Alkylen-Gruppe ist.

2. Verbindungen gemäß Anspruch 1, worin p = 0 ist und Ar eine Phenylen-Gruppe ist.

3. Verbindungen gemäß irgendeinem der Ansprüche 1 und 2, worin Z H, 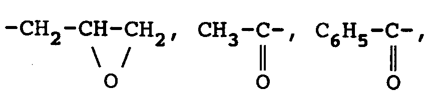 oder eine Allylgruppe ist.

4. Verbindungen gemäß irgendeinem der Ansprüche 1 bis 3, worin n etwa 20 ist.

5. Verbindungen gemäß irgendeinem der Ansprüche 1 bis 3, worin n etwa 90 ist.

6. Natrium-para-(hydroxy-polyethylenoxy)-benzoat der Formel 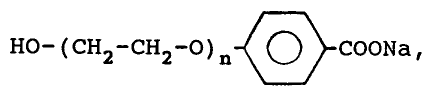 worin n etwa 90 ist.

7. Natrium-para-(glycidyloxy-polyethylenoxy)-benzoat der Formel 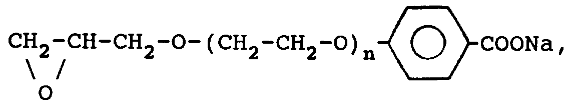 worin n etwa 90 ist.

8. Natrium-para-(acetoxy-polyethylenoxy)-benzoat der Formel 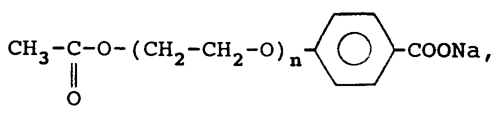 worin n etwa 90 ist.

9. Natrium-para-(benzyloxy-polyethylenoxy)-benzoat der Formel 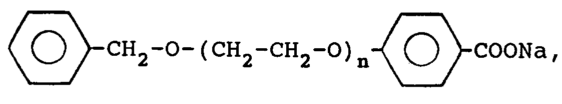 worin n etwa 90 ist.

10. Natrium-para-(hydroxy-polyethylenoxy)-benzoat der Formel 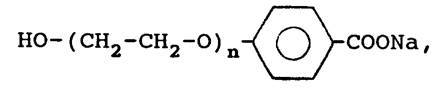 worin n etwa 20 ist.

## Patentansprüche (Patentansprüche für folgende(n) Vertragsstaat(en): ES)

1. Verfahren zur Herstellung von Verbindungen der allgemeinen Formel (I): 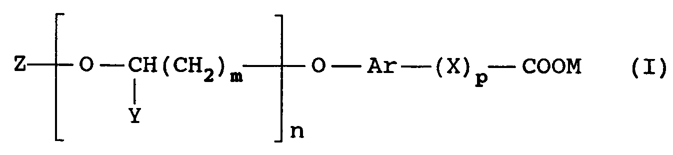 worin
M ein Alkalimetall oder ein halbes Äquivalent eines Erdalkalimetalls oder eine C₁-C₁₈-Alkyl-Gruppe ist;
X eine Gruppe ist, die ausgewählt ist aus -CH₂-, 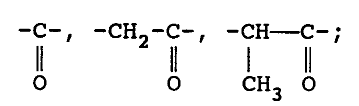
p = 0 oder 1 ist;
Ar ein einzelner oder ein kondensierter aromatischer Ring mit 6 bis 14 Kohlenstoffatomen ist, der wahlweise mit einer oder mehreren Gruppen substituiert ist, die ausgewählt sind aus Halogen-Atomen, C₁-C₈-Alkyl-, C₁-C₈-Alkoxy- oder Nitro-Gruppen; oder Ar für 2 oder mehrere aromatische Kerne steht, die entweder durch eine einfache kovalente Bindung oder durch eine -O-, -S-, -SO₂-, -SO-, -CO-, -CS- oder -NH- Gruppe miteinander verbunden sind;
Y H oder CH₃ ist;
m = 1 ist, wenn Y = CH₃ ist; oder eine ganze Zahl von 1 bis einschließlich 5 ist, wenn Y = H ist;
n eine Zahl im Bereich von 1 bis einschließlich 500 ist;
Z ausgewählt ist aus H; C₁-C₁₈-Alkyl, Benzyl, Allyl, 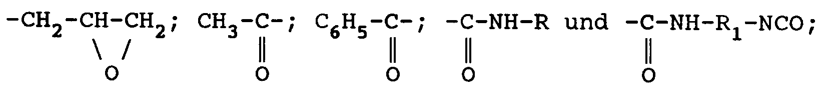 worin R eine unsubstituierte Phenyl-Gruppe ist oder eine Phenyl-Gruppe, die mit einem oder mehreren Halogen-Atomen substituiert ist, oder eine C₁₀-C₄₀-Alkyl-Gruppe ist; und worin R₁ unsubstituiertes Phenylen oder Phenylen bedeutet, welches mit einem oder mehreren Halogen-Atomen substituiert ist, oder eine C₁₀-C₄₀-Alkylen-Gruppe ist, dadurch gekennzeichnet, daß ein Alkyl-Ester einer Säure der Formel (III):
HO-Ar-(X)ₚ-COOH (III)
worin Ar, X und p die oben angegebenen Bedeutungen haben, in Gegenwart von Alkali bei einer Temperatur von 130 °C und bei einem Druck von bis zu 3,04 bar (3 Atmosphären) mit einem Alklylenoxid umgesetzt wird, und das so erhaltene Produkt, falls gewünscht, anschließend mit einem Metallhydroxid zum Salz dieser Säure verseift wird, worauf, falls gewünscht, die OH-Endgruppe der Polyoxyalkylen-Kette funktionalisiert wird.

2. Verfahren gemäß Anspruch 1, worin p = 0 ist und Ar eine Phenylen-Gruppe ist.

3. Verfahren gemäß irgendeinem der Ansprüche 1 und 2, worin Z H, 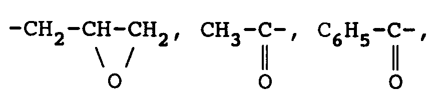 oder eine Allylgruppe ist.

4. Verfahren gemäß irgendeinem der Ansprüche 1 bis 3, worin n etwa 20 ist.

5. Verfahren gemäß irgendeinem der Ansprüche 1 bis 3, worin n etwa 90 ist.

6. Verfahren gemäß irgendeinem der Ansprüche 1 bis 5 zur Herstellung von Natrium-para-(hydroxy-polyethylenoxy)-benzoat der Formel 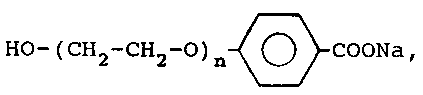 worin n etwa 90 ist.

7. Verfahren gemäß irgendeinem der Ansprüche 1 bis 5 zur Herstellung von Natrium-para-(glycidyloxy-polyethylenoxy)-benzoat der Formel 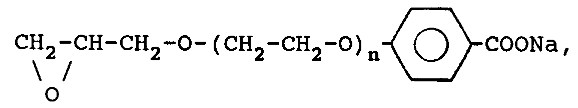 worin n etwa 90 ist.

8. Verfahren gemäß irgendeinem der Ansprüche 1 bis 5 zur Herstellung von Natrium-para-(acetoxy-polyethylenoxy)-benzoat der Formel 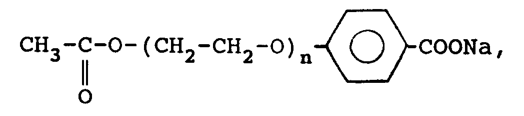 worin n etwa 90 ist.

9. Verfahren gemäß irgendeinem der Ansprüche 1 bis 5 zur Herstellung von Natrium-para-(benzyloxy-polyethylenoxy)-benzoat der Formel 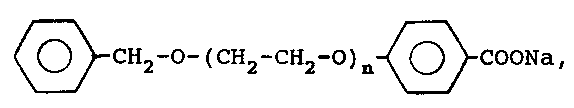 worin n etwa 90 ist.

10. Verfahren gemäß irgendeinem der Ansprüche 1 bis 5 zur Herstellung von Natrium-para-(hydroxy-polyethylenoxy)-benzoat der Formel 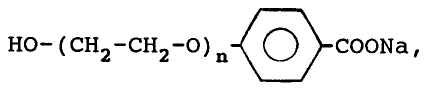 worin n etwa 20 ist.

## Revendications (Revendications pour l'(les) Etat(s) contractant(s) suivant(s): AT, BE, DE, FR, GB, NL, SE)

1. Composés ayant la formule générale (I) 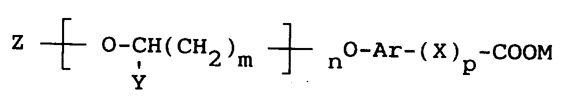 dans laquelle:
M est un métal alcalin ou la moitié d'un équivalent d'un métal alcalino-terreux ou un groupe alkyle en C₁₋₁₈;
X est un groupe choisi parmi -CH₂-, 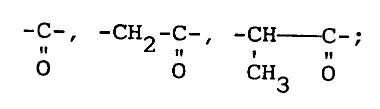
p est 0 ou 1;
Ar est un cycle aromatique unique ou condensé ayant de 6 à 14 atomes de carbone, éventuellement substitué avec un ou plusieurs atomes ou groupes choisis parmi les atomes d'halogène, les groupes alkyles en C₁₋₁₈, alcoxy en C₁₋₈ ou nitro; ou bien Ar représente deux ou plusieurs noyaux aromatiques, liés soit par une liaison covalente simple, soit par un groupe -O-, -S-, -SO₂-, -SO-, -CO-, -CS-, -NH-;
Y est un atome d'hydrogène ou un groupe CH₃;
m est 1 lorsque Y est un groupe CH₃ ou est un entier de 1 à 5 inclus lorsque Y est un atome d'hydrogène;
n est un nombre compris dans la gamme de 1 à 500 inclus;
Z est choisi parmi un atome d'hydrogène; un groupe alkyle en C₁₋₁₈, benzyle, allyle, 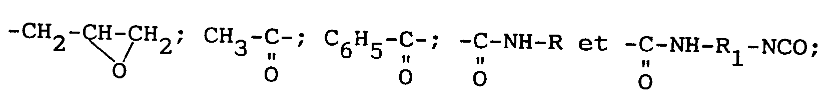 où R est un groupe phényle non substitué ou substitué
avec un ou plusieurs atomes d'halogène ou est un groupe alkyle en C₁₀₋₄₀; et R₁ représente un groupe phénylène non substitué ou un groupe phénylène substitué avec un ou plusieurs atomes d'halogène ou est un groupe alkylène en C₁₀₋₄₀.

2. Composés suivant la revendication 1, dans lesquels p est 0 et Ar est un groupe phénylène.

3. Composés suivant l'une quelconque des revendications 1 et 2, dans lesquels Z est un atome d'hydrogène, un groupe allyle, un groupe

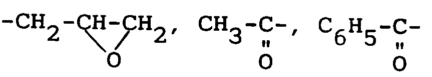

4. Composés suivant l'une quelconque des revendications 1 à 3, dans lesquels n est d'environ 20.

5. Composés suivant l'une quelconque des revendications 1 à 3, dans lesquels n est d'environ 90.

6. Para-(hydroxy-polyéthylèneoxy)-benzoate de sodium de formule 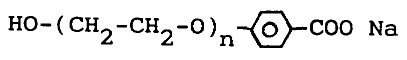 dans laquelle n est d'environ 90.

7. Para-(glycidoxy-polyéthylèneoxy)-benzoate de sodium de formule 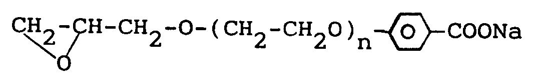 dans laquelle n est d'environ 90.

8. Para-(acétyloxy-polyéthylèneoxy)-benzoate de sodium de formule 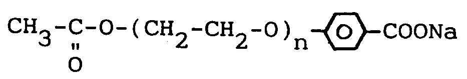 dans laquelle n est d'environ 90.

9. Para-(benzyloxy-polyéthylèneoxy)-benzoate de sodium de formule 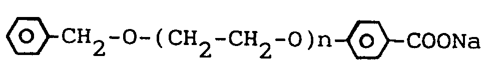 dans laquelle n est d'environ 90.

10. Para-(hydroxy-polyéthylèneoxy)-benzoate de sodium de formule 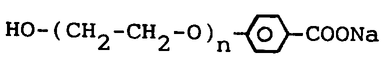 dans laquelle n est d'environ 20.

## Revendications (Revendications pour l'(les) Etat(s) contractant(s) suivant(s): ES)

1. Procédé pour la préparation de composés ayant la formule générale (I) 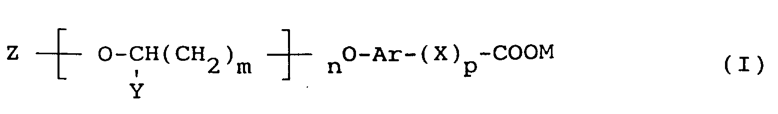 dans laquelle :
M est un métal alcalin ou la moitié d'un équivalent d'un métal alcalino-terreux, ou un groupe alkyle en C₁₋₁₈;
X est un groupe choisi parmi -CH₂-, 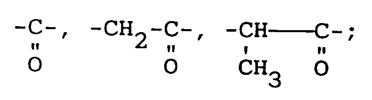
p est 0 ou 1;
Ar est un cycle aromatique unique ou condensé ayant de 6 à 14 atomes de carbone, éventuellement substitué avec un ou plusieurs atomes ou groupes choisis parmi les atomes d'halogène, les groupes alkyles en C₁₋₈, alcoxy en C₁₋₈ ou nitro; ou bien Ar représente deux ou plusieurs noyaux aromatiques, liés soit par une liaison covalente simple, soit par un groupe -O-, -S-, -SO₂-, -SO-, -CO-, -CS-, -NH-;
Y est un atome d'hydrogène ou un groupe CH₃;
m est 1 lorsque Y est un groupe CH₃ ou est un entier de 1 à 5 inclus lorsque Y est un atome d'hydrogène;
n est un nombre compris dans la gamme de 1 à 500 inclus;
Z est choisi parmi un atome d'hydrogène; un groupe alkyle en C₁₋₁₈, benzyle, allyle, 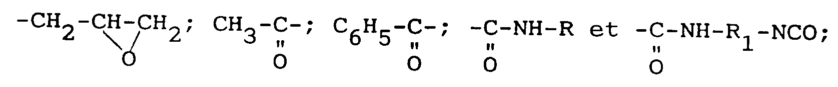 où R est un groupe phényle non substitué ou substitué
avec un ou plusieurs atomes d'halogène ou est un groupe alkyle en C₁₀₋₄₀; et R₁ représente un groupe phénylène non substitué ou un groupe phénylène substitué avec un ou plusieurs atomes d'halogène ou est un groupe alkylène en C₁₀₋₄₀, caractérisé en qu'un ester alkylique d'un acide ayant la formule (III):
HO - Ar - (X)ₚ - COOH (III)
dans laquelle Ar, X et p sont tels que définis plus haut, est amené à réagir avec un oxyde d'alkylène en présence d'un alcali à une température de 130°C et à une pression allant jusqu'à environ 3,04 bar (3 atmosphères), le produit ainsi obtenu est saponifié, si cela est désiré, avec un hydroxyde de métal pour obtenir le sel de l'acide, et le groupe terminal OH de chaîne polyoxyalkylène est ensuite fonctionalisé, si cela est désiré.

2. Procédé suivant la revendication 1, dans lequel p est 0 et Ar est un groupe phénylène.

3. Procédé suivant l'une quelconque des revendications 1 et 2, dans lequel Z est un atome d'hydrogène, un groupe allyle ou

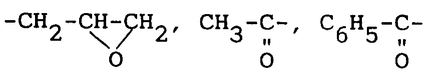

4. Procédé suivant l'une quelconque des revendications 1 à 3, dans lequel n est d'environ 20.

5. Procédé suivant l'une quelconque des revendications 1 à 3, dans lequel n est d'environ 90.

6. Procédé suivant l'une quelconque des revendications 1 à 5 pour la préparation du p-(hydroxy-polyéthylèneoxy)-benzoate de sodium de formule 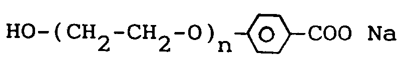 dans laquelle n est d'environ 90.

7. Procédé suivant l'une quelconque des revendications 1 à 5 pour la préparation du p-(glycidoxy-polyéthylèneoxy)-benzoate de sodium de formule 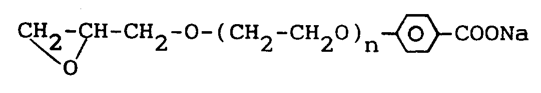 dans laquelle n est d'environ 90.

8. Procédé suivant l'une quelconque des revendications 1 à 5 pour la préparation du p-(acétyloxy-polyéthylèneoxy)-benzoate de sodium de formule 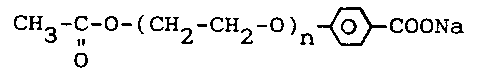 dans laquelle n est d'environ 90.

9. Procédé suivant l'une quelconque des revendications 1 à 5 pour la préparation du p-(benzyloxy-polyéthylèneoxy)-benzoate de sodium de formule 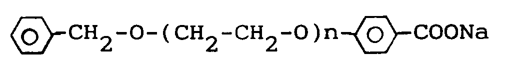 dans laquelle n est d'environ 90.

10. Procédé suivant l'une quelconque des revendications 1 à 5 pour la préparation du p-(hydroxy-polyéthylèneoxy)-benzoate de sodium de formule 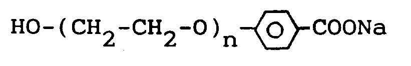 dans laquelle n est d'environ 20.
